# EUROPÄISCHE PATENTANMELDUNG

(11) **EP 2 540 293 A1**
(43) Veröffentlichungstag der Anmeldung: **02.01.2013**
(21) Anmeldenummer: 12167250.5
(22) Anmeldetag: 09.05.2012
(51) Int. Cl.: A61K 31/337, A61K 31/436, A61K 33/06, A61L 31/02, A61P 7/02, A61P 9/10

(54) **Erhöhung der effizienz der therapeutischen Wirkung pharmakologischer Wirsktoff-freissetzender medizinischer Vorrichtungen**

(30) Priorität: 28.06.2011 US 201161501794 P
(71) Anmelder: Biotronik AG, 8180 Bülach (CH)
(72) Erfinder: Sager, Laura, 8038 Zürich (CH); Klocke, Björn, 8006 Zürich (CH); Gratz, Matthias, 91054 Erlangen (DE)
(74) Vertreter: Lindner-Vogt, Karin L.

(57) **Zusammenfassung**

Die vorliegende Erfindung betrifft eine therapeutisch wirksame Zusammensetzung zur Behandlung von Arteriosklerose sowie eine pharmakologische Wirkstoff-freisetzende medizinische Vorrichtung, deren Effizienz durch Kombination mit einer erdalkalimetallionenfreisetzenden Verbindung erhöht wird.

## Beschreibung

Die vorliegende Erfindung betrifft eine therapeutisch wirksame Zusammensetzung zur Behandlung von Arteriosklerose sowie eine pharmakologische Wirkstoff-freisetzende medizinische Vorrichtung, welche diese therapeutisch wirksame Zusammensetzung aufweist, wobei die Effizienz der therapeutischen Wirkung dieser Wirkstoffe durch Kombination einer in wässrigem Milieu erdalkalimetallionenfreisetzenden Verbindung mit einem pharmakologischen Wirkstoff erhöht wird.

Eine der häufigsten Todesursachen in der entwickelten Welt sind kardiovaskuläre Erkrankungen, wobei Koronarerkrankungen von höchster Bedeutung sind. Zur Behandlung dieser Erkrankungen werden intravaskulär Gefäßprothesen, wie zum Beispiel Ballons oder Stents, in das betroffene Blutgefäß eines Patienten eingebracht und gegebenenfalls implantiert, um dieses aufzuweiten und offen zu halten.

Der Einsatz von Stents oder anderen medizinischen Vorrichtungen initiiert allerdings eine Kaskade von mikrobiologischen Prozessen, die beispielsweise eine Entzündung (Inflammation) des behandelten Hohlorgans oder eine nekrotische Veränderung begünstigen und die zu einem allmählichen Zuwachsen bspw. des Stents durch Bildung von Plaques führen können. Im schlimmsten Fall kann diese Veränderung des Hohlorgans eine erneute Verengung (Restenose) des Hohlorgans- bis hin zum Verschluss - bedingen.

Es ist wünschenswert, dass die eine Inflammation begünstigende Wirkung von medizinischen Vorrichtungen in Zukunft weitestgehend vermieden wird, da hierdurch die Wirksamkeit der medizinischen Vorrichtung verringert wird und weitere Schädigungen des behandelten Organismus hervorgerufen werden können.

Um die Risikofaktoren einer Restenose zu verhindern, wurde eine Vielzahl an Beschichtungen für Ballone und Stents entwickelt, die eine erhöhte Hämokompatibilität bieten sollen. Seit längerer Zeit werden in die Beschichtung von Ballonen und Stents antikoagulierende, antimikrobielle, antiinflammatorische und antiproliferative Agenzien einzeln oder in Kombination eingesetzt. Diese Substanzen sollen aus dem Beschichtungsmaterial des Ballons oder Stents derart freigesetzt werden, dass sie Entzündungen des umliegenden Gewebes, überschießendes Wachstum der glatten Muskelzellen oder das Verklumpen von Blut verhindern.

Ein zu erst viel versprechender Wirkstoff ihm Rahmen der Restenose-Prophylaxe ist Paclitaxel und Derivate hiervon. Diese bioverträgliche Substanz eignet sich insbesondere für den therapeutischen Einsatz zur Minderung überschießender Zellproliferation in der Gefäßwand nach Ballondilatation oder Stentimplantation, da sie auf Grund ihres lipophilen Charakters schnell in die Gefäßwand aufgenommen wird und lange wirksam ist.

Allerdings hat Paclitaxel einen sehr nachteiligen Einfluss zum einen auf die Erweiterung der Blutgefäße (Vasodilatation), bedingt durch die Minderung der Stickstoffmonoxid (NO) Verfügbarkeit, und zum anderen auf die Heilungseigenschaften der gereizten Gefäßwand, bedingt unter anderem durch die Inhibierung des Neointimawachstums. Diese Effekte führen zu einem deutlich erhöhten Thromboserisiko.

Aufgabe der vorliegenden Erfindung ist es somit, eine therapeutisch wirksame Zusammensetzung sowie eine medizinische Vorrichtung, welche diese therapeutisch wirksame Zusammensetzung aufweist, bereitzustellen, die durch Kombination einer in wässrigem Milieu erdalkalimetallionenfreisetzenden Verbindung mit einem pharmakologischen Wirkstoff die Effizienz der therapeutischen Wirkung dieser Wirkstoffe in Bezug auf die Beeinflussung der Vitalität, Viabilität, Proliferation, Migration und/oder Differenzierung von Zellen der Gefäßwand und angrenzender Gewebe erhöht.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Gemäß der vorliegenden Erfindung wird eine therapeutisch wirksame Zusammensetzung bestehend aus oder enthaltend mindestens einen pharmakologischen Wirkstoff und mindestens eine in wässrigem Milieu magnesiumionenfreisetzende Verbindung bereitgestellt.

Ein weiterer Gegenstand der vorliegenden Erfindung umfasst eine medizinische Vorrichtung, die eine therapeutisch wirksame Zusammensetzung bestehend aus oder enthaltend mindestens einen pharmakologischen Wirkstoff und mindestens eine in wässrigem Milieu magnesiumionenfreisetzende Verbindung aufweist.

Ein weiterer Gegenstand der vorliegenden Erfindung umfasst die Verwendung der erfindungsgemäßen therapeutisch wirksamen Zusammensetzung für die Herstellung einer medizinischen Vorrichtung.

Ein weiterer Gegenstand der vorliegenden Erfindung umfasst die Verwendung der erfindungsgemäßen therapeutisch wirksamen Zusammensetzung zur Prophylaxe oder Therapie einer Restenose oder einer Gefäßlumenbeeinträchtigung in einem Gefäßabschnitt.

Es hat sich überraschenderweise gezeigt, dass die erfindungsgemäße therapeutisch wirksame Zusammensetzung durch Kombination einer in wässrigem Milieu magnesiumionenfreisetzenden Verbindung mit einem pharmazeutischen Wirkstoff, mit Wirkung auf Vitalität, Viabilität, Proliferation, Migration und/oder Differenzierung von Zellen der Gefäßwand, einschließlich eingewanderter Immunzellen, und/oder angrenzender Gewebe, die therapeutische Wirkung dieser Wirkstoffe aufgrund zelltypspezifischer Effekte von Magnesiumionen auf vorgenannte Zellaktivitäten deutlich erhöht.

Unter der therapeutischen Wirkung im Rahmen der vorliegenden Erfindung versteht man die Summe der biologischen, chemischen und physiologischen Prozesse, die durch das Wirken des pharmakologischen Wirkstoffs hervorgerufen werden.

Der mindestens eine pharmakologische Wirkstoff ist ausgewählt aus der Gruppe der Taxane, insbesondere Paclitaxel und Derivate hiervon; Sirolimus und Derivate hiervon, insbesondere Biolimus A9, Everolimus, Zotarolimus, Myolimus, Novolimus, Temsirolimus; mTOR-Inhibitoren sowie Substanzen aus der Gruppe der Epothilone oder Derivate hiervon.

Die pharmakologischen Wirkstoffe können einzeln oder in Kombination, in gleicher oder unterschiedlicher Konzentration in der therapeutisch wirksamen Zusammensetzung vorliegen. Der mindestens eine pharmakologische Wirkstoff liegt bevorzugt in einer Konzentration von 0,0001-75 Gew.%, besonders bevorzugt von 0,001-25 Gew.% in der erfindungsgemäßen Zusammensetzung vor. Der pharmakologische Wirkstoff kann erfindungsgemäß auch als Prodrug in der Zusammensetzung vorliegen. Unter einem Prodrug versteht man eine pharmakologisch inaktive oder wenig aktive Verbindung, die erst im Organismus in den jeweiligen aktiven pharmakologischen Wirkstoff überführt wird, wie z.B. das Isotaxel, dass erst im Organismus zu Paclitaxel umgeformt wird.

Die in wässrigem Milieu magnesiumionenfreisetzende Verbindung ist ausgewählt aus der Gruppe Magnesiumoxid, Magnesiumhydroxid, Magnesiumcarbonat, Magnesiumaspartat und Derivate hiervon, bspw. Magnesiumbis(hydrogen-DL-aspartat), Magnesium-L-diglutamat, Magnesiumhydrogenphosphat, Magnesiumcitrat, Magnesiumsulfat, Magnesiumacetat, Magnesiumchlorid, Magnesiumfumarate, Magnesiumgluconat, Magnesiumglycinat, Magnesiumlactat, Magnesiumsalicylate und Magnesiumstearat. Besonders bevorzugt ist die in wässrigem Milieu magnesiumfreisetzende Verbindung ausgewählt aus Magnesiumhydroxid und Magnesiumsulfat. Die in wässrigem Milieu magnesiumfreisetzenden Verbindungen können einzeln oder in Kombination, in gleicher oder unterschiedlicher Konzentration in der therapeutisch wirksamen Zusammensetzung vorliegen. Das mindestens eine magnesiumionenfreisetzende Verbindung liegt bevorzugt in einer Konzentration von 25-99,9999 Gew.%, besonders bevorzugt von 75-99,9990 Gew.% in der erfindungsgemäßen Zusammensetzung vor.

Ein besonderer Vorteil der Verwendung einer in wässrigem Milieu magnesiumionenfreisetzenden Verbindung ist die ausgezeichnete Bioverträglichkeit. In wässrigem Milieu magnesiumionenfreisetzende Verbindungen, wie z.B. Magnesiumsalze, gehören zu den essentiellen Stoffen in der humanen Ernährung und sind für den Organismus unentbehrlich. Magnesium muss dem Körper über die Nahrung oder Nahrungsergänzungsmittel zugeführt werden, damit eine Vielzahl von biologischen, chemischen und physiologischen Prozessen im Organismus ohne Einschränkung ablaufen können, da Magnesiumionen u.a. für die katalytische Aktivität vieler essentieller Enzyme benötigt wird.

In einer besonders bevorzugten Ausführungsform enthält die therapeutisch wirksame Zusammensetzung als pharmakologischen Wirkstoff Paclitaxel oder Sirolimus und als eine in wässrigem Milieu magnesiumfreisetzende Verbindung Magnesiumhydroxid oder Magnesiumsulfat.

In einer bevorzugten Ausführungsform kann die therapeutisch wirksame Zusammensetzung optional weitere Zusatzstoffe ausgewählt aus der Gruppe Sorbitol, Titanoxid, Natriumdodecylsulfat, Natriumhydrogencarbonat, Kaliumhydrogencarbonat, Glycerin und Derivate hiervon, Harze wie Schellack, sowie Kontrastmittel, bspw. Iopromid (Ultravist®), enthalten, die die Verfügbarkeit der erfindungsgemäßen, pharmazeutischen Zusammensetzung am Wirkort positiv zu steuern in der Lage sind, also beispielsweise die Zeit der Verfügbarkeit verlängern, die Gewebeverteilung steuern, oder die maximal verfügbare Konzentration erhöhen können.

Weiterhin kann die Zusammensetzung als Lösung enthaltend den pharmakologischen Wirkstoff und die in wässrigem Milieu magnesiumfreisetzende Verbindung sowie optional weitere Zusatzstoffe vorliegen. Besonders geeignete Lösungsmittel sind Alkohole, bspw. Ethanol; und Aceton sowie Butyryl-trihexylcitrat.

Ein weiterer Gegenstand der vorliegenden Erfindung umfasst eine medizinische Vorrichtung, die eine therapeutisch wirksame Zusammensetzung bestehend aus oder enthaltend mindestens einen pharmakologischen Wirkstoff und mindestens eine in wässrigem Milieu magnesiumfreisetzende Verbindung aufweist, wobei als pharmakologischer Wirkstoff insbesondere Paclitaxel oder Sirolimus und als eine in wässrigem Milieu magnesiumfreisetzende Verbindung insbesondere Magnesiumhydroxid oder Magnesiumsulfat bevorzugt ist.

In einer bevorzugten Ausführungsform hiervon liegt die therapeutisch wirksame Zusammensetzung ganz oder in Teilen beschichtet auf der medizinischen Vorrichtung vor.

Eine Beschichtung im Sinne der Erfindung ist eine zumindest abschnittsweise Auftragung der Komponenten auf den Grundkörper der medizinischen Vorrichtung. Vorzugsweise wird die gesamte Oberfläche des Grundkörpers der medizinischen Vorrichtung von der Beschichtung bedeckt. Eine Schichtdicke liegt vorzugsweise im Bereich von 1 µm bis 100 µm, vorzugsweise besonders bevorzugt 3 µm bis 15 µm.

In einer besonders bevorzugten Ausführungsform liegt die therapeutisch wirksame Zusammensetzung in einer polymeren Trägermatrix vor.

Die polymere Trägermatrix im Rahmen der vorliegenden Erfindung ist zusammengesetzt aus Polymeren ausgewählt aus der Gruppe bestehend aus nichtresorbierbaren, permanenten Polymeren und/oder resorbierbaren bioabbaubaren Polymeren.

Besonders bevorzugt ist die polymere Trägermatrix zusammengesetzt aus Polymeren ausgewählt aus der Gruppe Polyolefine, Polyetherketone, Polyether, Polyvinylalkohole, Polyvinylhalogenide, Polyvinylester, Polyacrylate, Polyhalogenolefine, Polyamide, Polyamidimide, Polysulfone, Polyester, Polyurethane, Silikone, Polyphosphazene, Polyphenylen, Polymerschäume (aus Styrolen und Carbonaten), Polydioxanone, Polyglycolide, Polylactide, Poly-ε-caprolacton, Ethylvinylacetat, Polyethylenoxid, Polyphosphorylcholin, Polyhydroxybuttersäuren, Lipide, Polysaccharide, Proteine, Polypeptide sowie Copolymere, Blends und Derivate dieser Verbindungen.

Ganz besonders bevorzugt ist die polymere Trägermatrix zusammengesetzt aus Polymeren ausgewählt aus der Gruppe bestehend aus Polypropylen, Polyethylen, Polyisobutylen, Polybutylen, Polyetheretherketon, Polyethylenglycol, Polypropylenglycol, Polyvinylalkohole, Polyvinylchlorid, Polyvinylfluorid, Polyvinylacetat, Polyethylacrylat, Polymethylacrylat, Polytetrafluorethylen, Polychlortrifluorethylen, PA 11, PA 12, PA 46, PA 66, Polyamidimide, Polyethersulfon, Polyphenylsulfon, Polycarbonate, Polybutylenterephthalat, Polyethylenterephthalat, Elastane, Pellethane, Silikone, Polyphosphazene, Polyphenylen, Polymerschäume (aus Styrolen und Carbonaten), Polydioxanone, Polyglycolide, Poly-L-, Poly-D-, und Poly-D,L-Lactid, PLGA, sowie Poly-ε-caprolacton, Ethylvinylacetat, Polyethylenoxid, Polyphosphorylcholin, Polyhydroxyvalerat, Cholesterin, Cholesterinester, Alginat, Chitosan, Levan, Hyaluronsäure, Uronide, Heparin, Dextran, Cellulose, Fibrin, Albumin, Polypeptide und Copolymere, Blends und Derivate dieser Verbindungen.

Die Wahl der polymeren Trägermatrix richtet sich bevorzugt nach der gewünschten Elutionsgeschwindigkeit sowie den individuellen Charakteristika der verschiedenen eingesetzten Wirkstoffe und nach der unterschiedlichen Resorptions- bzw. Degradationsgeschwindigkeit am Wirkort des Medizinproduktes.

Der Gewichtsanteil einer erfindungsgemäßen polymeren Trägermatrix an den die Beschichtung bildenden Komponenten der Beschichtung beträgt vorzugsweise mindestens 40%, besonders bevorzugt mindestens 70%. Der Gewichtsanteil des mindestens einen pharmakologischen Wirkstoffs und der mindestens einen magnesiumionenfreisetzenden Verbindung an den die Beschichtung bildenden Komponenten der Beschichtung beträgt bevorzugt nicht mehr als 30%, besonders bevorzugt nicht mehr als 15%.

Die Beschichtung der gegebenenfalls in einer polymeren Trägermatrix vorliegenden erfindungsgemäßen Zusammensetzung kann direkt auf die medizinische Vorrichtung aufgetragen werden. Die Verarbeitung kann nach Standardverfahren für die Beschichtung erfolgen. Es können einschichtige, aber auch mehrschichtige Systeme (zum Beispiel so genannte base coat-, drug coat- oder top coat-Schichten) erstellt werden. Die Beschichtung kann unmittelbar auf dem Grundkörper der Vorrichtung aufgebracht sein oder es sind weitere Schichten dazwischen vorgesehen, die beispielsweise der Haftvermittlung dienen.

In einer weiteren bevorzugten Ausführungsform kann der mindestens eine pharmakologische Wirkstoff und die mindestens eine in wässrigem Milieu magnesiumionenfreisetzende Verbindung aus unterschiedlichen polymeren Trägermatrices abgegeben werden. Medizinische Vorrichtungen innerhalb des Schutzumfangs der vorliegenden Erfindungen beinhalten beliebige medizinische Produkte, welche benutzt werden, wenigstens teilweise, um in den Körper eines Patienten eingebracht zu werden. Beispiele umfassen Tabletten, Kapseln, Stents, Wirkstoffpumpen, Kanülen, Spritzen, Herzschrittmacher, Katheter, Nadelinjektionskatheter, vaskuläre Transplantate, Ballons, Organe, Gefäße, Aorten, Herzklappen, Schläuche, Organersatzteile, Fasern, Hohlfasern, Membranen, Konserven, Blutbehältem, Dialysatoren sowie Sensoren. Insbesondere bevorzugt handelt es sich bei der medizinischen Vorrichtung um einen Ballon oder einen Stent.

Bei einem Ballon gemäß der vorliegenden Erfindung handelt es sich um dem Fachmann bekannte Ballone oder Ballonkatheter, die in der perkutanen transluminalen Angioplastie (PTA) oder perkutanen transluminalen Coronarangioplastie (PTCA) Verwendung finden. Diese gegebenenfalls Mehrfachballone werden hergestellt aus Materialien wie Polyester, Polyolefine, Nylon, Polyurethane, Fluorpolymere, Polyamide, Polyethylenterephthalat (PET), Polybutylenterephthalat (PBT) oder Polyether-Polyurethane sowie deren Copolymere und Blends. Bevorzugt ist der Ballon aus PEBAX hergestellt.

Bei einem Stent gemäß der vorliegenden Erfindung handelt es sich um einen biodegradierbaren oder permanenten, selbstexpandierbaren oder ballonexpandierbaren Stent.

Stents herkömmlicher Bauart weisen eine filigrane Stützstruktur aus metallischen Streben auf, die zur Einbringung in den Körper zunächst in einem nicht-expandierten Zustand vorliegen und die am Ort der Applikation dann in einen expandierten Zustand aufgeweitet werden. Der Stent kann vor oder nach dem Crimpen auf einen Ballon beschichtet werden.

Der Grundkörper des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink, Platin, Iridium oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung. In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol.

In einem weiteren bevorzugten Ausführungsbeispiel besteht der Grundkörper des Stents aus Edelstahl, vorzugsweise aus einem Cr-Ni-Fe-Stahl- hier bevorzugt die Legierung 316L - oder einem Co-Cr-Stahl. Ferner kann der Grundkörper des Stents mindestens teilweise aus Kunststoff und/oder einer Keramik bestehen.

Zusätzlich kann auf dem Grundkörper des Stents bestehend aus einem metallischen Material eine passivierende Siliziumkarbid-Schicht (SiC) vorgesehen sein. Diese wird nach dem Fachmann bekannten Verfahren aufgebracht und befindet sich unter der Schicht enthaltend die erfindungsgemäße Zusammensetzung.

In einem weiteren Ausführungsbeispiel besteht der Grundkörper des Stents aus einem biokorrodierbaren metallischen Werkstoff, zum Beispiel einer biokorrodierbaren Legierung, ausgewählt aus der Gruppe Magnesium, Eisen und Wolfram; insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. In einer weiteren bevorzugten Magnesiumlegierung liegt der Rest < 5 Gew.% und umfasst beispielsweise 0,4 - 1,0 Gew.% Zirkonium und 2,0 - 2,5 Gew.% Neodym. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

In einem weiteren Ausführungsbeispiel besteht der Stent aus natürlichen Polymeren, wie zum Beispiel aus Collagen, Chitin, Chitosan, Heparin. In einem weiteren Ausführungsbeispiel besteht der Stent aus abbaubaren Polymeren, wie zum Beispiel aus einem Polylactid wie PDLA, PLLA, PLGA, sowie P3HB, P4HB oder Caprolacton und Copolymeren aus den genannten Polymeren.

Das Stentdesign sollte vorzugsweise derart angepasst sein, dass ein möglichst großer Kontakt zur Gefäßwand besteht. Dies begünstigt eine gleichmäßige Elution des pharmakologischen Wirkstoffs.

Alternativ kann die Beschichtung aus der erfindungsgemäßen Zusammensetzung als Kavitätenfüllung vorliegen oder Bestandteil einer Kavitätenfüllung sein. Die medizinische Vorrichtung, insbesondere der Ballon oder der Stent, weist zu diesem Zweck eine oder mehrere Kavitäten auf. Kavitäten befinden sich beispielsweise an der Oberfläche der medizinischen Vorrichtung und lassen sich je nach Vorrichtung nach dem Fachmann bekannte Verfahren erstellen. Der Fachmann kann sich bei der Ausgestaltung der Kavität an dem im Stand der Technik beschriebenen Systemen orientieren. Der Begriff "Kavität" umfasst dabei z.B. Löcher und Vertiefungen.

In einer besonders bevorzugten Ausführungsform weist die medizinische Vorrichtung, insbesondere ein Ballon oder ein biodegradierbarer oder permanenter, selbstexpandierbarer oder ballonexpandierbarer Stent, eine therapeutisch wirksame Zusammensetzung auf, die als pharmakologischen Wirkstoff Paclitaxel oder Sirolimus und als eine in wässrigem Milieu magnesiumfreisetzende Verbindung Magnesiumhydroxid oder Magnesiumsulfat sowie optional weitere, oben definierte, Zusatzstoffe enthält. Dabei kann der pharmakologische Wirkstoff Paclitaxel oder Sirolimus und/oder die in wässrigem Milieu magnesiumfreisetzende Verbindung Magnesiumhydroxid oder Magnesiumsulfat und/oder die optional weiteren Zusatzstoffe in der gleichen oder unterschiedlichen polymeren Trägermatrices vorliegen.

Dabei enthält die therapeutisch wirksame Zusammensetzung Paclitaxel oder Sirolimus in Mengen von 5 bis 30 mg, bevorzugt 15 bis 25 mg, und Magnesiumsulfat oder Magnesiumhydroxid in Mengen von 50 bis 900 mg, bevorzugt 250 bis 600 mg. Als polymere Trägermatrix wird bevorzugt Poly-L-lactid, Poly-DL-lactid oder PGLA verwendet.

Weiterhin kann die Zusammensetzung als Lösung, enthaltend den pharmakologischen Wirkstoff und die in wässrigem Milieu magnesiumfreisetzende Verbindung sowie optional weitere Zusatzstoffe, vorliegen und anschließend auf die medizinische Vorrichtung beschichtet werden. Dabei können die jeweiligen Komponenten in der gleichen oder unterschiedlichen polymeren Trägermatrices vorliegen. Besonders geeignete Lösungsmittel sind Alkohole, bspw. Ethanol; und Aceton.

Magnesiumionen haben eine Eigenwirkung auf die Proliferation glatter Muskelzellen: sie reduzieren diese mehr als die Proliferation von Endothelzellen. Aufgrund der unterschiedlichen Wirkmechanismen von Paclitaxel oder Sirolimus in Kombination mit einer in wässrigem Milieu magnesiumfreisetzenden Verbindung, erfolgt eine additive Wirkverstärkung der Kombination.

Ein weiterer Gegenstand der vorliegenden Erfindung ist die Verwendung einer therapeutisch wirksamen Zusammensetzung bestehend aus oder enthaltend mindestens einen pharmakologischen Wirkstoff und mindestens eine in wässrigem Milieu magnesiumfreisetzende Verbindung, für die Herstellung einer medizinischen Vorrichtung.

Ein weiterer Gegenstand der vorliegenden Erfindung ist die Verwendung einer therapeutisch wirksamen Zusammensetzung bestehend aus oder enthaltend mindestens einen pharmakologischen Wirkstoff und mindestens eine in wässrigem Milieu magnesiumfreisetzende Verbindung, zur Prophylaxe oder Therapie einer Restenose oder einer Gefäßlumenbeeinträchtigung in einem Gefäßabschnitt.

### Ausführungsbeispiel 1

30 mg Paclitaxel werden in Ethanol gelöst und mit Macrogolglycerolricinoleat versetzt. Anschließend werden 900mg Magnesiumsulfat zugegeben und gelöst.

### Ausführungsbeispiel 2

30 mg Paclitaxel werden in Ethanol gelöst und mit Iopromid versetzt. Anschließend werden 900mg Magnesiumsulfat zugegeben und gelöst.

### Ausführungsbeispiel 3

30 mg Paclitaxel werden in Ethanol gelöst und mit Macrogolglycerolricinoleat versetzt. Anschließend werden 900mg Magnesiumhydroxid zugegeben und gelöst.

### Ausführungsbeispiel 4

30 mg Paclitaxel werden in Ethanol gelöst und mit Iopromid versetzt. Anschließend werden 900mg Magnesiumhydroxid zugegeben und gelöst.

### Ausführungsbeispiel 5

30 mg Paclitaxel werden in Aceton enthaltend PLGA gelöst und mit 900mg Magnesiumhydroxid versetzt.

Medizinische Vorrichtungen, bspw. Tabletten, Kapseln, Stents, Wirkstoffpumpen, Kanülen, Spritzen, Herzschrittmacher, Katheter, Nadelinjektionskatheter, vaskuläre Transplantate, Ballons, Organe, Gefäße, Aorten, Herzklappen, Schläuche, Organersatzteile, Fasern, Hohlfasern, Membranen, Konserven, Blutbehältern, Dialysatoren sowie Sensoren, insbesondere ein Ballon oder ein biodegradierbarer oder permanenter, selbstexpandierbarer oder ballonexpandierbarer Stent können die oben aufgeführten erfindungsgemäßen Zusammensetzungen (Ausführungsbeispiele 1 bis 5) aufweisen.

Beispielsweise kann die erfindungsgemäße Zusammensetzung nach einem der Ausführungsbeispiele 1 bis 4 über eine Spritze oder eine Kanüle systemisch verabreicht werden, über eine Dauer von einer halben bis drei Stunden oder auch für einen oder mehreren Tage, bspw. 90 Tage.

Weiterhin kann die erfindungsgemäße Zusammensetzung nach einem der Ausführungsbeispiele 1 bis 5 auf einen biodegradierbaren oder permanenten, selbstexpandierbaren oder ballonexpandierbaren Stent aufgebracht werden. Hierbei werden dem Fachmann bekannte Methoden verwendet, die erfindungsgemäße Zusammensetzung auf die Oberfläche des Stents aufzubringen und gegebenenfalls zu trocknen. So kann die Beschichtung beispielsweise mittels Tauchen oder Sprühen auf die Oberfläche aufgebracht und bei 40°C unter Vakuum getrocknet werden. Bevorzugt weist die Oberfläche des Stents eine Konzentration an Paclitaxel und magnesiumionenfreisetzender Verbindung von 2.5 bis 10.0 µg/mm² Stentoberfläche auf.

## Patentansprüche

1. Therapeutisch wirksame Zusammensetzung bestehend aus oder enthaltend mindestens einen pharmakologischen Wirkstoff und mindestens eine in wässrigem Milieu magnesiumionenfreisetzende Verbindung.

2. Therapeutisch wirksame Zusammensetzung nach Anspruch 1, wobei der mindestens eine pharmakologische Wirkstoff ausgewählt ist aus der Gruppe der Taxane, insbesondere Paclitaxel und Derivate hiervon; Sirolimus und Derivate hiervon, insbesondere Biolimus A9, Everolimus, Zotarolimus, Myolimus, Novolimus, Temsirolimus; mTOR-Inhibitoren sowie Substanzen aus der Gruppe der Epothilone oder Derivate hiervon.

3. Therapeutisch wirksame Zusammensetzung nach Anspruch 1 oder 2, wobei die mindestens eine in wässrigem Milieu magnesiumionenfreisetzende Verbindung ausgewählt ist aus der Gruppe Magnesiumoxid, Magnesiumhydroxid, Magnesiumcarbonat, Magnesiumaspartat und Derivate hiervon, Magnesium-L-diglutamat, Magnesiumhydrogenphosphat, Magnesiumcitrat, Magnesiumsulfat, Magnesiumacetat, Magnesiumchlorid, Magnesiumfumarate, Magnesiumgluconat, Magnesiumglycinat, Magnesiumlactat, Magnesiumsalicylate und Magnesiumstearat.

4. Therapeutisch wirksame Zusammensetzung nach einem der vorhergehenden Ansprüche, wobei der pharmakologische Wirkstoff Paclitaxel und die magnesiumionenfreisetzende Verbindung Magnesiumhydroxid oder Magnesiumsulfat ist.

5. Therapeutisch wirksame Zusammensetzung nach einem der Ansprüche 1 bis 3, wobei der pharmakologische Wirkstoff Sirolimus und die magnesiumionenfreisetzende Verbindung Magnesiumhydroxid oder Magnesiumsulfat ist.

6. Therapeutisch wirksame Zusammensetzung nach einem der vorhergehenden Ansprüche, wobei die therapeutisch wirksame Zusammensetzung optional weitere Zusatzstoffe ausgewählt aus der Gruppe Sorbitol, Titanoxid, Natriumdodecylsulphat, Natriumhydrogencarbonat, Kaliumhydrogencarbonat, Glycerin und Derivate hiervon, sowie Kontrastmittel enthält.

7. Medizinische Vorrichtung, **dadurch gekennzeichnet, dass** sie eine therapeutisch wirksame Zusammensetzung nach einem der Ansprüche 1 bis 6 aufweist.

8. Medizinische Vorrichtung nach Anspruch 7, wobei die therapeutisch wirksame Zusammensetzung ganz oder in Teilen beschichtet auf der medizinischen Vorrichtung vorliegt und gegebenenfalls in einer polymeren Trägermatrix vorliegt.

9. Medizinische Vorrichtung nach Anspruch 8, wobei der mindestens eine pharmakologische Wirkstoff und die mindestens eine in wässrigem Milieu magnesiumionenfreisetzenden Verbindung aus unterschiedlichen polymeren Trägermatrices abgegeben werden.

10. Medizinische Vorrichtung nach einem der Ansprüche 7 bis 9, wobei die Vorrichtung ausgewählt ist aus der Gruppe bestehend aus Tabletten, Kapseln, Stents, Wirkstoffpumpen, Kanülen, Spritzen, Herzschrittmacher, Katheter, Nadelinjektionskatheter, vaskuläre Transplantate, Ballons, Organe, Gefäße, Aorten, Herzklappen, Schläuche, Organersatzteile, Fasern, Hohlfasern, Membranen, Konserven, Blutbehältern, Dialysatoren sowie Sensoren.

11. Medizinische Vorrichtung nach Anspruch 10, wobei die Vorrichtung ein Ballon oder ein Stent ist.

12. Medizinische Vorrichtung nach Anspruch 11, wobei der Stent ein biodegradierbarer oder permanenter, selbstexpandierbarer oder ballonexpandierbarer Stent ist.

13. Medizinische Vorrichtung nach einem der Ansprüche 7 bis 12, wobei die therapeutisch wirksame Zusammensetzung als pharmakologischen Wirkstoff Paclitaxel und als magnesiumionenfreisetzende Verbindung Magnesiumhydroxid oder Magnesiumsulfat sowie optional weitere Zusatzstoffe aufweist.

14. Medizinische Vorrichtung nach Anspruch 13, wobei der pharmakologische Wirkstoff Paclitaxel und/oder die magnesiumionenfreisetzende Verbindung Magnesiumhydroxid oder Magnesiumsulfat und/oder die optional weiteren Zusatzstoffe in der gleichen oder unterschiedlichen polymeren Trägermatrices vorliegen.

15. Verwendung einer therapeutisch wirksamen Zusammensetzung nach einem der Ansprüche 1 bis 6 zur Prophylaxe oder Therapie einer Restenose oder einer Gefäßlumenbeeinträchtigung in einem Gefäßabschnitt.
